(19) Europäisches Patentamt
European Patent Office
Office européen des brevets

(11) EP 1 960 440 B1

(12) **EUROPÄISCHE PATENTSCHRIFT**

(45) Veröffentlichungstag und Bekanntmachung des Hinweises auf die Patenterteilung:
**11.01.2017 Patentblatt 2017/02**

(21) Anmeldenummer: **06819855.5**

(22) Anmeldetag: **30.11.2006**

(51) Int Cl.:
***C08F 8/00*** *(2006.01)* ***A61L 15/60*** *(2006.01)*

(86) Internationale Anmeldenummer:
**PCT/EP2006/069088**

(87) Internationale Veröffentlichungsnummer:
**WO 2007/065834 (14.06.2007 Gazette 2007/24)**

(54) **VERFAHREN ZUR HERSTELLUNG WASSERABSORBIERENDER POLYMERE MIT HOHER ABSORPTIONSKAPAZITÄT UND HOHER PERMEABILITÄT**

PROCESS FOR PREPARING WATER-ABSORBING POLYMERS WITH HIGH ABSORPTION CAPACITY AND HIGH PERMEABILITY

PROCEDE DE FABRICATION D'UN POLYMERE ABSORBANT L'EAU PRESENTANT UNE GRANDE CAPACITE D'ABSORPTION ET UNE GRANDE PERMEABILITE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **05.12.2005 US 742458 P**

(43) Veröffentlichungstag der Anmeldung:
**27.08.2008 Patentblatt 2008/35**

(73) Patentinhaber: **BASF SE**
**67056 Ludwigshafen am Rhein (DE)**

(72) Erfinder:
- **WENDKER, Martin**
**67549 Worms (DE)**
- **HERFERT, Norbert**
**63674 Altenstadt (DE)**
- **MCCORMACK, Paul Walter**
**67433 Neustadt (DE)**

(74) Vertreter: **BASF IP Association**
**BASF SE**
**ZRX-C6**
**67056 Ludwigshafen (DE)**

(56) Entgegenhaltungen:
**EP-A- 1 357 133**
**WO-A-2004/024816**
**WO-A-2005/080479**
**US-A1- 2002 128 618**
**US-A1- 2004 176 544**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 960 440 B1

## Beschreibung

Die vorliegende Erfindung betrifft Verfahren zur Herstellung wasserabsorbierender Polymere mit hoher Absorptionskapazität und hoher Permeabilität, die wasserabsorbierenden Polymere sowie Hygieneartikel, die diese enthalten.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Herstellung wasserabsorbierender Polymere ist vielfach beschrieben, siehe beispielsweise "Modern Superabsorbent Polymer Technology", F.L. Buchholz and A.T. Graham, Wiley-VCH, 1998, Seiten 69 bis 117.

Wasserabsorbierende Polymere haben typischerweise eine Zentrifugenretentionskapazität von 25 bis 60 g/g, vorzugsweise von mindestens 30 g/g, bevorzugt von mindestens 32 g/g, besonders bevorzugt von mindestens 34 g/g, ganz besonders bevorzugt von mindestens 35 g/g. Die Zentrifugenretentionskapazität (CRC) wird gemäß der von der EDANA (European Disposables and Nonwovens Association) empfohlenen Testmethode Nr. 441.2-02 "Centrifuge retention capacity" bestimmt.

Zur Verbesserung der Anwendungseigenschaften, wie beispielsweise Permeabilität, werden wasserabsorbierende Polymere im allgemeinen nachvernetzt. Diese Nachvernetzung kann in wässriger Gelphase durchgeführt werden. Vorzugsweise werden aber gemahlene und abgesiebte Polymerpartikel (Grundpolymer) an der Oberfläche mit einem Nachvernetzer beschichtet, getrocknet und thermisch nachvernetzt. Dazu geeignete Vernetzer sind Verbindungen, die mindestens zwei Gruppen enthalten, die mit den Carboxylatgruppen des hydrophilen Polymeren kovalente Bindungen bilden können oder die mindestens zwei Carboxylgruppen oder andere funktionelle Gruppen mindestens zweier verschiedener Polymerketten des Grundpolymers miteinander vernetzen können.

Durch die Nachvernetzung sinkt die Absorptionskapazität der wasserabsorbierenden Polymere, d.h. Absorptionskapazität und Permeabilität sind gegenläufige Eigenschaften.

EP-A 372 981 offenbart wasserabsorbierende Polymere mit verbesserter Absorption und Haltbarkeit im nassen Zustand.

EP-A 574 260 beschreibt ein Verfahren zur Herstellung wasserabsorbierender Polymere mit weniger Restmonomeren.

US 5,684,1 06, WO2004/024816 und WO2005/080479 lehren die Herstellung wasserabsorbierender Polymere mit verbessertem Eigenschaftsprofil.

WO 00/22018 offenbart wasserabsorbierende Polymere mit hohem Absorptionsvermögen und verbesserter Kapillarität.

WO 00/53644, WO 00/53664, WO 02/20068 und WO 02/22717 beschreiben wasserabsorbierende Polymere mit verbesserten Eigenschaften, insbesondere Retention, Rückhaltevermögen und Transport von Flüssigkeiten.

Die Aufgabe der vorliegenden Erfindung war die Bereitstellung wasserabsorbierender Polymere mit hoher Absorptionskapazität und hoher Permeabilität.

Gelöst wurde die Aufgabe durch ein Verfahren zur Herstellung wasserabsorbierender Polymere durch Polymerisation einer Monomerlösung, enthaltend

a) mindestens ein ethylenisch ungesättigtes, säuregruppentragendes Monomer, das zumindest teilweise neutralisiert sein kann,
b) 0,01 bis 0,5 Gew.-% mindestens einen Vernetzer, bezogen auf das Monomer a),
c) wahlweise ein oder mehrere mit den unter a) genannten Monomeren copolymerisierbare ethylenisch und/oder allylisch ungesättigte Monomere und
d) wahlweise ein oder mehrere wasserlösliche Polymere,

wobei die Monomerlösung polymerisiert und das erhaltene Polymer mit

e) 0,05 bis 0,25 Gew.-% mindestens eines Nachvernetzers, bezogen auf das Polymer, wobei der Nachvernetzer kein Polyol ist, und
f) 0,05 bis 0,5 Gew.-% mindestens eines mehrwertigen Kations, bezogen auf das Polymer,

nachbehandelt wird.

Der vorliegenden Erfindung liegt die Erkenntnis zugrunde, dass sich Absorptionskapazität und Permeabilität unter bestimmten Bedingungen nahezu additiv verhalten. Durch Verwendung geringerer Mengen an Vernetzer b) und

Nachvernetzer e) werden wasserabsorbierende Polymere mit hoher Absorptionskapazität erhalten. Die Nachbehandlung mit dem mehrwertigen Kation f) führt zu einer Erhöhung der Permeabilität, ohne den sonst üblichen deutlichen Abfall der Absorptionskapazität.

Grundsätzlich sind auch Polyole als Nachvernetzer bekannt. Allerdings führt die Verwendung von Polyolen als Nachvernetzer zu einem unerwünscht starken Abfall der Absorptionskapazität.

Die Nachbehandlung mit dem Nachvernetzer e) und dem mehrwertigen Kation f) kann in einem gemeinsamen Verfahrensschritt erfolgen. Dabei ist es möglich den Nachvernetzer e) und das mehrwertige Kation f) als gemeinsame Lösung zu dosieren. Vorteilhaft ist aber die getrennte Dosierung.

Es ist aber auch möglich den Nachvernetzer e) und das mehrwertige Kation f) in getrennten Verfahrensschritten zu dosieren. Dabei wird vorteilhaft das mehrwertige Kation f) in dem nachfolgenden Verfahrensschritt zugesetzt.

Geeignete Monomere a) sind beispielsweise ethylenisch ungesättigte Carbonsäuren, wie Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden

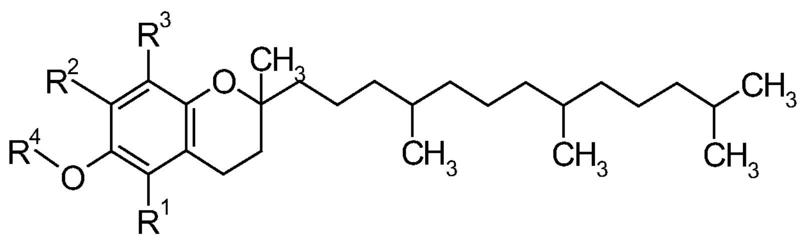

wobei $R^1$ Wasserstoff oder Methyl, $R^2$ Wasserstoff oder Methyl, $R^3$ Wasserstoff oder Methyl und $R^4$ Wasserstoff oder ein Säurerest mit 1 bis 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit $R^1 = R^2 = R^3$ = Methyl, insbesondere racemisches alpha-Tocopherol. $R^4$ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere bevorzugt um 50 Gew.-ppm, Hydrochinonhalbether, jeweils bezogen auf Acrylsäure, wobei Acrylsäuresalze rechnerisch als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die wasserabsorbierenden Polymere sind vernetzt, d.h. die Polymerisation wird in Gegenwart von Verbindungen mit mindestens zwei polymerisierbaren Gruppen, die in das Polymernetzwerk radikalisch einpolymerisiert werden können, durchgeführt. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A 530 438 beschrieben, Di- und Triacrylate, wie in EP-A 547 847, EP-A 559 476, EP-A 632 068, WO 93/21237, WO 03/104299, WO 03/104300, WO 03/104301 und DE-A 103 31 450 beschrieben, gemischte Acrylate, die neben Acrylatgruppen weitere ethylenisch ungesättigte Gruppen enthalten, wie in DE-A 103 31 456 und DE-A 103 55 401 beschrieben, oder Vernetzermischungen, wie beispielsweise in DE-A 195 43 368, DE-A 196 46 484, WO 90/15830 und WO 02/32962 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A 343 427 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 20-fach ethoxylierten Glyzerins,

des 3- bis 20-fach ethoxylierten Trimethylolpropans, des 3- bis 20-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des mindestens 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in DE-A 103 19 462 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3-bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Die Menge an Vernetzer b) beträgt vorzugsweise 0,05 bis 0,4 Gew.-%, besonders bevorzugt 0,1 bis 0,3 Gew.-%, jeweils bezogen auf das Monomer a).

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die Herstellung eines geeigneten Polymers sowie weitere geeignete hydrophile ethylenisch ungesättigte Monomere a) werden in DE-A 199 41 423, EP-A 686 650,
WO 01/45758 und WO 03/104300 beschrieben.

Geeignete Reaktoren sind Knetreaktoren oder Bandreaktoren. Im Kneter wird das bei der Polymerisation einer wässrigen Monomerlösung entstehende Polymergel durch beispielsweise gegenläufige Rührwellen kontinuierlich zerkleinert, wie in WO 01/38402 beschrieben. Die Polymerisation auf dem Band wird beispielsweise in DE-A 38 25 366 und US 6,241,928 beschrieben. Bei der Polymerisation in einem Bandreaktor entsteht ein Polymergel, das in einem weiteren Verfahrensschritt zerkleinert werden muss, beispielsweise in einem Fleischwolf, Extruder oder Kneter.

Vorteilhaft wird das Hydrogel nach dem Verlassen des Polymerisationsreaktors noch bei höherer Temperatur, vorzugsweise mindestens 50°C, besonders bevorzugt mindestes 70°C, ganz besonders bevorzugt mindestens 80°C, sowie vorzugsweise weniger als 100°C, gelagert, beispielsweise in isolierten Behältern. Durch die Lagerung, üblicherweise 2 bis 12 Stunden, wird der Monomerumsatz weiter erhöht.

Die Säuregruppen der erhaltenen Hydrogele sind üblicherweise teilweise neutralisiert, vorzugsweise zu 25 bis 95 mol-%, bevorzugt zu 50 bis 80 mol-%, besonders bevorzugt zu 60 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen.

Die Neutralisation wird vorzugsweise auf der Stufe der Monomeren durchgeführt. Dies geschieht üblicherweise durch Einmischung des Neutralisationsmittels als wässrige Lösung, als Schmelze, oder bevorzugt auch als Feststoff. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Es ist aber auch möglich die Neutralisation nach der Polymerisation auf der Stufe des Hydrogels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Hydrogels eingestellt wird. Wird das Hydrogel zumindest teilweise nach der Polymerisation neutralisiert, so wird das Hydrogel vorzugsweise mechanisch zerkleinert, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden.

Das Hydrogel wird dann vorzugsweise mit einem Bandtrockner getrocknet bis der Restfeuchtegehalt vorzugsweise unter 15 Gew.-%, insbesondere unter 10 Gew.-% liegt, wobei der Wassergehalt gemäß der von der EDANA (European Disposables and Nonwovens Association) empfohlenen Testmethode Nr. 430.2-02 "Moisture content" bestimmt wird. Wahlweise kann zur Trocknung aber auch ein Wirbelbetttrockner oder ein beheizter Pflugscharmischer verwendet werden. Um besonders weiße Produkte zu erhalten, ist es vorteilhaft bei der Trocknung dieses Gels einen schnellen Abtransport des verdampfenden Wassers sicherzustellen. Dazu ist die Trocknertemperatur zu optimieren,

die Luftzu- und -abführung muss kontrolliert erfolgen, und es ist in jedem Fall auf ausreichende Belüftung zu achten. Die Trocknung ist naturgemäß umso einfacher und das Produkt umso weißer, wenn der Feststoffgehalt des Gels möglichst hoch ist. Bevorzugt liegt der Feststoffgehalt des Gels vor der Trocknung daher zwischen 30 und 80 Gew.-%. Besonders vorteilhaft ist die Belüftung des Trockners mit Stickstoff oder einem anderen nicht-oxidierenden Inertgas. Wahlweise kann aber auch einfach nur der Partialdruck des Sauerstoffs während der Trocknung abgesenkt werden, um oxidative Vergilbungsvorgänge zu verhindern. Im Regelfall führt aber auch eine ausreichende Belüftung und Abführung des Wasserdampfes zu einem noch akzeptablen Produkt. Vorteilhaft hinsichtlich Farbe und Produktqualität ist in der Regel eine möglichst kurze Trocknungszeit.

Eine weitere wichtige Funktion der Trocknung des Gels ist die hier noch stattfindende Verringerung des Restmonomerengehaltes im Superabsorber. Bei der Trocknung zerfallen nämlich eventuell noch vorhandene Reste der Initiatoren und führen zu einer Einpolymerisation von noch vorhandenen Restmonomeren. Außerdem reißen die verdampfenden Wassermengen noch vorhandene freie wasserdampfflüchtige Monomere, wie beispielsweise Acrylsäure mit, und verringern so ebenfalls den Restmonomerengehalt im Superabsorber.

Das getrocknete Hydrogel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen eingesetzt werden können.

Anschließend wird das erhaltene Polymer nachvernetzt. Hierzu geeignete Nachvernetzer e) sind Verbindungen, die mindestens zwei Gruppen enthalten, die mit den Carboxylatgruppen der Polymere kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A 083 022, EP-A 543 303 und EP-A 937 736 beschrieben, oder β-Hydroxyalkylamide, wie in DE-A 102 04 938 und US 6,239,230 beschrieben. Geeignet sind ferner Verbindungen mit gemischter Funktionalität, wie Glycidol, 3-Ethyl-3-oxetanmethanol (Trimethylolpropanoxetan), wie in EP-A 1 199 327 beschrieben, oder Verbindungen, die nach der ersten Reaktion eine weitere Funktionalität ausbilden, wie Ethylenoxid, Propylenoxid, Isobutylenoxid, Aziridin, Azetidin oder Oxetan.

Des weiteren sind in DE-A 40 20 780 zyklische Karbonate, in DE-A 198 07 502 2-Oxazolidon und dessen Derivate, wie N-(2-Hydroxyethyl)-2-oxazolidon, in DE-A 198 07 992 Bis- und Poly-2-oxazolidinone, in DE-A 198 54 573 2-Oxotetrahydro-1,3-oxazin und dessen Derivate, in DE-A 198 54 574 N-Acyl-2-oxazolidone, in DE-A 102 04 937 zyklische Harnstoffe, in DE-A 103 34 584 bizyklische Amidacetale, in EP-A 1 199 327 Oxetane und zyklische Harnstoffe und in WO 03/031482 Morpholin-2,3-dion und dessen Derivate als geeignete Nachvernetzer e) beschrieben.

Bevorzugte Nachvernetzer e) sind Oxazolidon und dessen Derivate, insbesondere N-(2-Hydroxyethyl)-2-oxazolidon.

Die Menge an Nachvernetzer e) beträgt vorzugsweise 0,08 bis 0,22 Gew.-%, besonders bevorzugt 0,1 bis 0,2 Gew.-%, jeweils bezogen auf das Polymer.

Die Nachvernetzer e) werden üblicherweise als wäßrige Lösung eingesetzt, vorzugsweise wird dabei zusätzlich ein Cosolvens verwendet. Technisch gut geeignete Cosolventien sind $C_1$-$C_6$-Alkohole, wie Methanol, Ethanol, n-Propanol, Isopropanol, n-Butanol, sec-Butanol, tert-Butanol oder 2-Methyl-1-Propanol, $C_2$-$C_5$-Diole, wie Ethylenglykol, 1,2-Propylenglykol, 1,3-Propandiol oder 1,4-Butandiol, oder Ketone, wie Aceton.

Bei Verwendung von Diolen als Cosolvens ist darauf zu achten, dass die Diole nicht oder unter nur in untergeordnetem Maß als Nachvernetzer wirken sollten. Aufgrund ihrer deutlich geringeren Reaktivität kann dies durch Wahl geeigneter Reaktionsbedingungen, wie Zeit und Temperatur, erreicht werden.

Die Nachvernetzung wird üblicherweise so durchgeführt, dass eine Lösung des Nachvernetzers e) auf das Hydrogel oder die trockenen Polymerpartikel aufgesprüht wird. Im Anschluss an das Aufsprühen wird thermisch getrocknet, wobei die Nachvernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige®-Mischer, Bepex®-Mischer, Nauta®-Mischer, Processall®-Mischer und Schugi®-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und Nara®-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 170 bis 250°C, bevorzugt 180 bis 220°C, und besonders bevorzugt 190 bis 210°C. Die bevorzugte Verweilzeit bei dieser Temperatur im Reaktionsmischer oder Trockner beträgt mindestens 45 Minuten, besonders bevorzugt mindestens 60 Minuten.

Die im erfindungsgemäßen Verfahren einsetzbaren mehrwertigen Kationen f) sind beispielsweise zweiwertige Kationen, wie die Kationen von Zink, Magnesium, Kalzium und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat ist bevorzugt.

Üblicherweise wird das mehrwertige Kation f) als wässrige Lösung eingesetzt. Die Konzentration des mehrwertigen Kations f) in der wässrigen Lösung beträgt beispielsweise 0,1 bis 12 Gew.-%, vorzugsweise 0,5 bis 8 Gew.-%, besonders bevorzugt 1,5 bis 4 Gew.-%.

Die Menge an mehrwertigem Kation f) beträgt vorzugsweise 0,06 bis 0,25 Gew.-%, besonders bevorzugt 0,07 bis 0,2 Gew.-%, ganz besonder bevorzugt 0,08 bis 0,15 Gew.-%, jeweils bezogen auf das Polymer.

Durch das erfindungsgemäße Verfahren können wasserabsorbierende Polymere mit einer Zentrifugenretentionskapazität (CRC) von mindestens 16 g/g, vorzugsweise 28 bis 50 g/g, besonders bevorzugt von 30 bis 40 g/g, ganz besonders bevorzugt von 31 bis 35 g/g, und einer Permeabilität (FS-FIP) von mindestens 16 $x10^{-6}$ $cm^3$ s/g, vorzugsweise von 17 bis 45 $x10^{-6}$ $cm^3$ s/g, besonders bevorzugt von 19 bis 35 $x10^{-6}$ $cm^3$ s/g, ganz besonders bevorzugt von 20 bis 28 $x10^{-6}$ $cm^3$ s/g, erhalten werden.

Weitere Gegenstände der vorliegenden Erfindung sind die gemäß dem erfindungsgemäßen Verfahren erhältlichen Polymere sowie Hygieneartikel, insbesondere Windeln, die diese enthalten.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Das Quellbare hydrogelbildende Polymer wird vor der Messung gut durchmischt.

Zentrifugenretentionskapazität (CRC Centrifuge Retention Capacity)

Die Zentrifugenretentionskapazität der wasserabsorbierenden Polymere wird gemäß der von der EDANA (European Disposables and Nonwovens Association) empfohlenen Testmethode Nr. 441.2-02 "Centrifuge retention capacity" bestimmt.

Flüssigkeitstransport (FS-FIP Free Swell Fixed Insult Permeability)

Bei dieser Methode wird die Durchlässigkeit einer gequollenen Gelschicht für eine bestimmte Menge an Flüssigkeit in Abhängigkeit der Zeit bestimmt.

Eine Abbildung der Testzelle findet sich in Abbildung 1. Darin bedeuten:

A äußere Zelle
B innere Zelle
C Gewicht
a Marke1
b Marke2

Die äußere Zelle A ist ein unten mit einem Sieb verschlossener Plexiglaszylinder mit einem Innendurchmesser von 2,55 cm.

Die innere Zelle B ist ein unten mit einem Sieb verschlossener Plexiglaszylinder mit einem Außendurchmesser von 2,45 cm. Im oberen Bereich der Zelle B befindet sich ein ringförmiges Metallgewicht C. Das Auflagegewicht der Zelle B mit dem Gewicht C beträgt 29 $g/cm^2$.

Zu Beginn des Tests wird die innere Zelle B in die äußere Zelle A eingesetzt und die Höhe der zusammengebauten Messzelle gemessen und als $h_0$ notiert. Anschließend wird die innere Zelle B wieder entfernt. Für den Test werden 0,160 g Superabsorber in die äußere Zelle A eingewogen.

Die präparierte äußere Zelle A wird auf eine Sinterplatte (Porosität 0), die sich wiederum in einer mit 0,9 gew.-%igen Natriumchloridlösung gefüllten Petrischale befindet, gestellt und das Testmaterial für eine Stunde gequollen. Anschließend wird die innere Zelle B mit dem Gewicht C wieder in die äußere Zelle A eingesetzt.

Im Folgenden wird die Gelschicht zweimal mit je 25 ml einer 0,9 gew.-%igen Natriumchlorid-Lösung gewaschen, wobei die Lösung über die innere Zelle B eingefüllt wird, und die Höhe der zusammengebauten Messzelle als $h_1$ gemessen.

Für die eigentliche Messung werden dreimal hintereinander je 25 ml der 0,9 gew.-%igen Natriumchlorid-Lösung auf das Gelbett gegeben, wiederum über die innere Zelle B, und jeweils die Zeiten als $t_1$ bis $t_3$ notiert, die der Flüssig-

keitsspiegel benötigt, um von Marke1 (6 cm oberhalb des Siebes) bis zur Marke2 (4 cm oberhalb des Siebes) der inneren Zelle B durchzufließen. Zum Abschluss wird nochmals die Höhe der zusammengebauten Messzelle als $h_2$ gemessen.

Die Permeabilität des gequollenen Gels kann nun nach Darcy's Law abgeleitet werden.

Dabei berechnet sich die durchschnittliche Gelhöhe [cm] nach

$$h_n = \frac{(h_2 - h_0) + (h_1 - h_o)}{2}$$

Die durchschnittliche Durchflusszeit [s] berechnet sich nach

$$t_n = \frac{t_1 + t_2 + t_3}{3}$$

Die durchschnittliche volumetrische Flussrate [$cm^3/s$] berechnet sich nach der folgenden Formel, wobei sich das Volumen 5,38 $cm^3$ aus den Zellenmaßen und dem Abstand von 2 cm der beiden Marken (4 und 6 cm) ergibt:

$$\frac{dV}{dt} = \frac{5{,}38cm^3}{t_n}$$

H als Durchschnittshöhe [cm] der Flüssigkeit über dem Boden des Gelbettes während der Messung wird nach der folgenden Formel berechnet:

$$H = h_n * \frac{Markel + Marke2}{2}$$

Die FS-FIP [$10^{-6}$ $cm^3$ s/g] wird nun nach Darcy's Law ermittelt:

$$FS\text{-}FIP = \frac{\frac{(dV)}{dt} * h_n}{A * P}$$

mit A als Oberfläche des Gelbettes ($\pi r^2$ = 5,107 $cm^2$) und mit dem durchschnittlichen hydrostatischen Druck P = pgH (Dichte p = 1 $g/cm^3$ und Fallbeschleunigung g = 981 $cm/s^2$).

Beispiele

Beispiel 1:

5.166 g einer 37,3 gew.-%igen Natriumacrylatlösung wurden mit 574 g Acrylsäure und 720 g Wasser gemischt und mit Stickstoff inertisiert. Diese Mischung wurde in einen mit Stickstoff inertisierten Kneter vom Typ Werner & Pfleiderer LUK 8,0 K2 (2 Sigma - Wellen) eingefüllt. Diese Mischung entspricht einem Neutralisationsgrad der Acrylsäure von 72 mol-%. Anschließend wurden nacheinander 14 g 15fach ethoxyliertes Trimethylolpropantriacrylat (entspricht 0,56 Gew.-% bezogen auf Acrylsäure), 10 g einer 0,75 gew.-%igen Ascorbinsäurelösung, 16,6 g einer 15 gew.-%igen Natriumpersulfatlösung und 3,75 g einer 3 gew.-%igen Wasserstoffperoxidlösung zugegeben. Der Kneter wurde bei Maximaldrehzahl (98 upm der schnelleren Welle, ca. 49 upm auf der langsameren Welle, Verhältnis ca. 2:1) gerührt. Sofort nach der Zugabe von Wasserstoffperoxid wurde der Knetermantel auf 80°C erwärmt. Nach Erreichen der Maximaltemperatur wurde der Knetermantel nicht weiter beheizt und weitere 15 Minuten nachreagieren lassen. Das Gel wurde auf 65°C abgekühlt und ausgefüllt. Es wurde ein Gel mit einigen ca. 1 bis 2 cm großen Gelklumpen erhalten.

Das Gel wurde bei 170°C für 75 Minuten mit einer Beladung von 700 g pro Blech im Umlufttrockenschrank

getrocknet. Nach dreimaligen Mahlen in einem Walzenstuhl (Gebr. Baumeister LRC 125/70, Spaltbreiten 1000 μm, 600 μm und 400 μm) wurde das Polymer auf einen Siebschnitt zwischen 850 und 100 μm abgesiebt.

Das hergestellte Polymer hatte eine Zentrifugenretentionskapazität (CRC) von 35,8 g/g.

Beispiel 2 (Vergleich)

Ein wie in Beispiel 1 hergestelltes Polymer mit einem Neutralisationsgrad der Acrylsäure von 72 mol-%, 0,56 Gew.-% eines 15fach ethoxylierten Trimethylolpropantriacrylats und einer CRC von 35,5 g/g wurde in einem Labor-Pflugscharmischer der Fa. Lödige mittels einer Zweistoffdüse mit einer wässrigen Oberflächennachvernetzer-Lösung (0,1 Gew.-% Ethylenglykoldiglycidylether, 2,3 Gew.-% Wasser, 1,0 Gew.-% 1,2-Propandiol) besprüht. Danach sprühte man 1,36 Gew.-% einer 22 gew.-%igen Aluminiumsulfat-Lösung auf und temperte bei 175°C für eine Stunde. Nach dem Abkühlen erfolgte eine Absiebung auf 106 bis 850 μm.

Beispiel 3 (Vergleich)

Ein wie in Beispiel 1 hergestelltes Polymer mit einem Neutralisationsgrad der Acrylsäure von 72 mol-%, 0,84 Gew.-% eines 15fach ethoxylierten Trimethylolpropantriacrylats und einer CRC von 32 g/g wurde in einem Labor-Pflugscharmischer der Fa. Lödige mittels einer Zweistoffdüse mit einer wässrigen Oberflächennachvernetzer-Lösung (0.12 Gew.-% Ethylenglykoldiglycidylether, 1,8 Gew.-% Wasser, 0,6 Gew.-% 1,2-Propandiol) besprüht. Danach sprühte man 2,45 Gew.-% einer 22 gew.-%igen Aluminiumsulfat-Lösung auf und temperte bei 180°C für eine Stunde. Nach dem Abkühlen erfolgte eine Absiebung auf 106 bis 850 μm.

Beispiel 4

Ein wie in Beispiel 1 hergestelltes Polymer mit einem Neutralisationsgrad der Acrylsäure von 72 mol-%, 0,44 Gew.-% 3fach ethoxiliertes Glycerintriacrylat und einer CRC von 36 g/g wurde in einem Labor-Pflugscharmischer der Fa. Lödige mittels einer Zweistoffdüse mit einer wässrigen Oberflächennachvernetzer-Lösung (0,12 Gew.-% Ethylenglykoldiglycidylether, 1,8 Gew.-% Wasser, 0,6 Gew.-% 1,2-Propandiol) besprüht. Danach sprühte man 2,45 Gew.-% einer 22 gew.-%igen Aluminiumsulfat-Lösung auf und temperte bei 175°C für eine Stunde. Nach dem Abkühlen erfolgte eine Absiebung auf 106 bis 850 μm.

Beispiel 5

Ein wie in Beispiel 1 hergestelltes Polymer mit einem Neutralisationsgrad der Acrylsäure von 75 mol-%, 0,21 Gew.-% Polyethylenglykoldiacrylat (ca. 9 Ethylenoxideinheiten) einer CRC von 39,9 g/g wurde in einem Labor-Pflugscharmischer der Fa. Lödige mittels einer Zweistoffdüse mit einer wässrigen Oberflächennachvernetzer-Lösung (0.08 Gew.-% Ethylenglykoldiglycidylether, 0,75 Gew.-% Wasser, 0,75 Gew.-% 1,2-Propandiol) besprüht. Danach sprühte man 2,45 Gew.-% einer 22 gew.-%igen Aluminiumsulfat-Lösung auf und temperte bei 180°C für eine Stunde. Nach dem Abkühlen erfolgte eine Absiebung auf 106 bis 850 μm.

Beispiel 6

Ein wie in Beispiel 1 hergestelltes Polymer mit einem Neutralisationsgrad der Acrylsäure von 75 mol-%, 0,18 Gew.-% 3fach ethoxyliertes Glycerintriacrylat und einer CRC von 40,2 g/g wurde in einem Labor-Pflugscharmischer der Fa. Lödige mittels einer Zweistoffdüse mit einer wässrigen Oberflächennachvernetzer-Lösung (0,1 Gew.-% N-(2-Hydroxyethyl)-2-oxazolidinon, 0,75 Gew.-% Wasser, 0,75 Gew.-% Isopropanol) besprüht. Danach sprühte man 2,45 Gew.-% einer 22 gew.-%igen Aluminiumsulfat-Lösung auf und temperte bei 190°C für eine Stunde. Nach dem Abkühlen erfolgte eine Absiebung auf 106 bis 850 μm.

Beispiel 7

Ein wie in Beispiel 1 hergestelltes Polymer mit einem Neutralisationsgrad der Acrylsäure von 77 mol-%, 0,18 Gew.-% 3fach ethoxyliertes Glycerintriacrylat und einer CRC von 41 g/g wurde in einem Labor-Pflugscharmischer der Fa. Lödige mittels einer Zweistoffdüse mit einer wässrigen Oberflächennachvernetzer-Lösung (0,08 Gew.-% Ethylenglykoldiglycidylether, 0,75 Gew.-% Wasser, 0,75 Gew.-% 1,2-Propandiol) besprüht. Danach sprühte man 2,45 Gew.-% einer 22 gew.-%igen Aluminiumsulfat-Lösung auf und temperte bei 170°C für eine Stunde. Nach dem Abkühlen auf 40-50°C wurden erneut 2,45 Gew.-% einer 22 gew.-%igen Aluminiumsulfat-Lösung aufgesprüht, noch 10 Minuten gemischt und abschließend bei 106 bis 850 μm abgesiebt.

Tab. 1:

| | FS-FIP [$10^{-6}$ $cm^3$ s/g] | CRC [g/g] |
|---|---|---|
| Beispiel 1 (Vgl.) | 0,83 | 35,8 |
| Beispiel 2 (Vgl.) | 11,68 | 28,0 |
| Beispiel 3 (Vgl.) | 27,34 | 23,4 |
| Beispiel 4 | 27,34 | 28,9 |
| Beispiel 5 | 17,93 | 33,7 |
| Beispiel 6 | 18,65 | 32,4 |
| Beispiel 7 | 24,16 | 33,1 |

**Patentansprüche**

**1.** Verfahren zur Herstellung wasserabsorbierender Polymere durch Polymerisation einer Monomerlösung, enthaltend

a) mindestens ein ethylenisch ungesättigtes, säuregruppentragendes Monomer, das zumindest teilweise neutralisiert sein kann,
b) 0,01 bis 0,5 Gew.-% mindestens eines Vernetzers, bezogen auf das Monomer a),
c) wahlweise ein oder mehrere mit den unter a) genannten Monomeren copolymerisierbare ethylenisch und/oder allylisch ungesättigte Monomere und
d) wahlweise ein oder mehrere wasserlösliche Polymere,

wobei die Monomerlösung polymerisiert und das erhaltene Polymer mit

e) 0,05 bis 0,25 Gew.-% mindestens eines Nachvernetzers, bezogen auf das Polymer, wobei der Nachvernetzer kein Polyol ist, und
f) 0,05 bis 0,5 Gew.-% mindestens eines mehrwertigen Kations, bezogen auf das Polymer,

nachbehandelt wird.

**2.** Verfahren gemäß Anspruch 1, **dadurch gekennzeichnet, dass** Nachvernetzer und mehrwertiges Kation in einem gemeinsamen Verfahrensschritt zugesetzt werden.

**3.** Verfahren gemäß Anspruch 1, **dadurch gekennzeichnet, dass** der Nachvernetzer vor dem mehrwertigen Kation zugesetzt wird.

**4.** Verfahren gemäß einem der Ansprüche 1 bis 3, **dadurch gekennzeichnet, dass** das Polymer während oder nach Zusatz des Nachvernetzers erwärmt wird.

**5.** Verfahren gemäß Anspruch 4, **dadurch gekennzeichnet, dass** das Polymer während oder nach Zusatz des Nachvernetzers und vor Zusatz des mehrwertigen Kations erwärmt wird.

**6.** Verfahren gemäß einem der Ansprüche 1 bis 5, **dadurch gekennzeichnet, dass** das Polymer nach Zusatz des Nachvernetzers auf mindestens 170°C erwärmt wird.

**7.** Verfahren gemäß einem der Ansprüche 1 bis 6, **dadurch gekennzeichnet, dass** das Polymer nach Zusatz des Nachvernetzers für mindestens 45 Minuten erwärmt wird.

**8.** Verfahren gemäß Anspruch 1 bis 7, **dadurch gekennzeichnet, dass** das ethylenisch ungesättigte, säuregruppentragende Monomer zu mindestens 60 mol-% neutralisiert ist.

**9.** Wasserabsorbierende Polymere, enthaltend

i) mindestens ein einpolymerisiertes ethylenisch ungesättigtes, säuregruppentragendes Monomer, das zumindest teilweise neutralisiert sein kann,
ii) 0,01 bis 0,5 Gew.-% mindestens eines einpolymerisierten Vernetzers, bezogen auf das Monomer a),
iii) wahlweise ein oder mehrere einpolymerisierte mit den unter i) genannten Monomeren copolymerisierbare ethylenisch und/oder allylisch ungesättigte Monomere,
iv) wahlweise ein oder mehrere wasserlösliche Polymere, auf die die unter i) genannten Monomere zumindest teilweise aufgepfropft sind,
v) 0,05 bis 0,25 Gew.-% mindestens eines einpolymerisierten Nachvernetzers, bezogen auf das Polymer, wobei der Nachvernetzer kein Polyol ist, und
vi) 0,05 bis 0,5 Gew.-% mindestens eines mehrwertigen Kations, bezogen auf das Polymer.

**10.** Polymer gemäß Anspruch 9, **dadurch gekennzeichnet, dass** das einpolymerisierte ethylenisch ungesättigte, säuregruppentragende Monomer zu mindesten 60 mol-% neutralisiert ist.

**11.** Polymer mit einer Zentrifugenretentionskapazität (CRC, gemessen nach EDANA-Testmethode Nr. 441.2-02) von mindestens 27 g/g und einer Permeabilität (FS-FIP, gemessen wie bei der Beschreibung der Methoden beschrieben) von mindestens 16 x $10^{-6}$ $cm^3$ s/g.

**12.** Hygieneartikel, enthaltend ein wasserabsorbierendes Polymer gemäß einem der Ansprüche 9 bis 11.

## Claims

**1.** A process for preparing water-absorbing polymers by polymerizing a monomer solution comprising

a) at least one ethylenically unsaturated, acid-bearing monomer which may be at least partly neutralized,
b) from 0.01 to 0.5% by weight of at least one crosslinker based on the monomer a),
c) if desired one or more ethylenically and/or allylically unsaturated monomers copolymerizable with the monomers mentioned under a) and
d) if desired one or more water-soluble polymers,

by polymerizing the monomer solution and aftertreating the resulting polymer with

e) from 0.05 to 0.25% by weight of at least one postcrosslinker, based on the polymer, where the crosslinker is not a polyol, and
f) from 0.05 to 0.5% by weight of at least one polyvalent cation based on the polymer.

**2.** The process according to claim 1, wherein postcrosslinker and polyvalent cation are added in a common process step.

**3.** The process according to claim 1, wherein the postcrosslinker is added before the polyvalent cation.

**4.** The process according to any of claims 1 to 3, wherein the polymer is heated during or after addition of the postcrosslinker.

**5.** The process according to claim 4, wherein the polymer is heated during or after addition of the postcrosslinker and before addition of the polyvalent cation.

**6.** The process according to any of claims 1 to 5, wherein the polymer is heated to at least 170°C after addition of the postcrosslinker.

**7.** The process according to any of claims 1 to 6, wherein the polymer is heated for at least 45 minutes after addition of the postcrosslinker.

**8.** The process according to claim 1 to 7, wherein the ethylenically unsaturated, acid-bearing monomer has been neutralized to an extent of at least 60 mol%.

**9.** A water-absorbing polymer comprising

i) at least one polymerized ethylenically unsaturated, acid-bearing monomer which may be at least partly neutralized,
ii) from 0.01 to 0.5% by weight of at least one polymerized crosslinker based on the monomer a),
iii) if desired one or more polymerized ethylenically and/or allylically unsaturated monomers copolymerizable with the monomers mentioned under i),
iv) if desired one or more water-soluble polymers to which the monomers mentioned under i) have at least partly been grafted,
v) from 0.05 to 0.25% by weight of at least one polymerized postcrosslinker, based on the polymer, where the crosslinker is not a polyol, and
vi) from 0.05 to 0.5% by weight of at least one polyvalent cation based on the polymer.

**10.** The polymer according to claim 9, wherein the polymerized ethylenically unsaturated, acid-bearing monomer has been neutralized to an extent of at least 60 mol%.

**11.** A polymer having a Centrifuge Retention Capacity (CRC, measured by EDANA test method No. 441.2-02) of at least 27 g/g and a permeability (FS-FIP, measured as described in the description of the methods) of at least 16 x $10^{-6}$ $cm^3$ s/g.

**12.** A hygiene article comprising a water-absorbing polymer according to any of claims 9 to 11.

**Revendications**

**1.** Procédé pour la préparation de polymères absorbant l'eau par polymérisation d'une solution de monomères, contenant

a) au moins un monomère éthyléniquement insaturé, portant des groupes acides, qui peut être au moins partiellement neutralisé,
b) 0,01 à 0,5% en poids d'au moins un réticulant, par rapport au monomère a),
c) éventuellement un ou plusieurs monomères éthyléniquement et/ou allyliquement insaturés copolymérisables avec les monomères cités en a), et
d) éventuellement un ou plusieurs polymères solubles dans l'eau,

la solution de monomères étant polymérisée et le polymère obtenu étant posttraité avec

e) 0,05 à 0,25% en poids d'au moins un postréticulant, par rapport au polymère, le postréticulant n'étant pas un polyol, et
f) 0,05 à 0,5% en poids d'au moins un cation polyvalent, par rapport au polymère.

**2.** Procédé selon la revendication 1, **caractérisé en ce que** le postréticulant et le cation polyvalent sont ajoutés dans une étape de procédé commune.

**3.** Procédé selon la revendication 1, **caractérisé en ce que** le postréticulant est ajouté avant le cation polyvalent.

**4.** Procédé selon l'une quelconque des revendications 1 à 3, **caractérisé en ce que** le polymère est chauffé pendant ou après l'addition du postréticulant.

**5.** Procédé selon la revendication 4, **caractérisé en ce que** le polymère est chauffé pendant ou après l'addition du postréticulant et avant l'addition du cation polyvalent.

**6.** Procédé selon l'une quelconque des revendications 1 à 5, **caractérisé en ce que** le polymère est chauffé à au moins 170°C après l'addition du postréticulant.

**7.** Procédé selon l'une quelconque des revendications 1 à 6, **caractérisé en ce que** le polymère est chauffé après l'addition du postréticulant pendant au moins 45 minutes.

**8.** Procédé selon la revendication 1 à 7, **caractérisé en ce que** le monomère éthyléniquement insaturé, portant des groupes acides, est neutralisé à raison d'au moins 60% en mole.

**9.** Polymères absorbant l'eau, contenant

i) au moins un monomère éthyléniquement insaturé copolymérisé, portant des groupes acides, qui peut être au moins partiellement neutralisé,
ii) 0,01 à 0,5% en poids d'au moins un réticulant copolymérisé, par rapport au monomère a),
iii) éventuellement un ou plusieurs monomères éthyléniquement insaturés et/ou allyliquement insaturés, copolymérisables avec les monomères cités en i), copolymérisés, et
iv) éventuellement un ou plusieurs polymères solubles dans l'eau sur lesquels les monomères mentionnés en i) sont au moins partiellement greffés,
v) 0,05 à 0,25% en poids d'au moins un postréticulant copolymérisé, par rapport au polymère, le postréticulant n'étant pas un polyol, et
vi) 0,05 à 0,5% en poids d'au moins un cation polyvalent, par rapport au polymère.

**10.** Polymère selon la revendication 9, **caractérisé en ce que** le monomère éthyléniquement insaturé, portant des groupes acides, copolymérisé, est neutralisé à raison d'au moins 60% en mole.

**11.** Polymère présentant une capacité de rétention à la centrifugeuse (CRC, mesurée selon le procédé de test EDANA n° 441.2-02) d'au moins 27 g/g et une perméabilité (FS-FIP, mesurée selon la description des procédés) d'au moins 16 x $10^{-6}$ $cm^3$ s/g.

**12.** Objets hygiéniques contenant un polymère absorbant l'eau selon l'une quelconque des revendications 9 à 11.

Abb. 1:

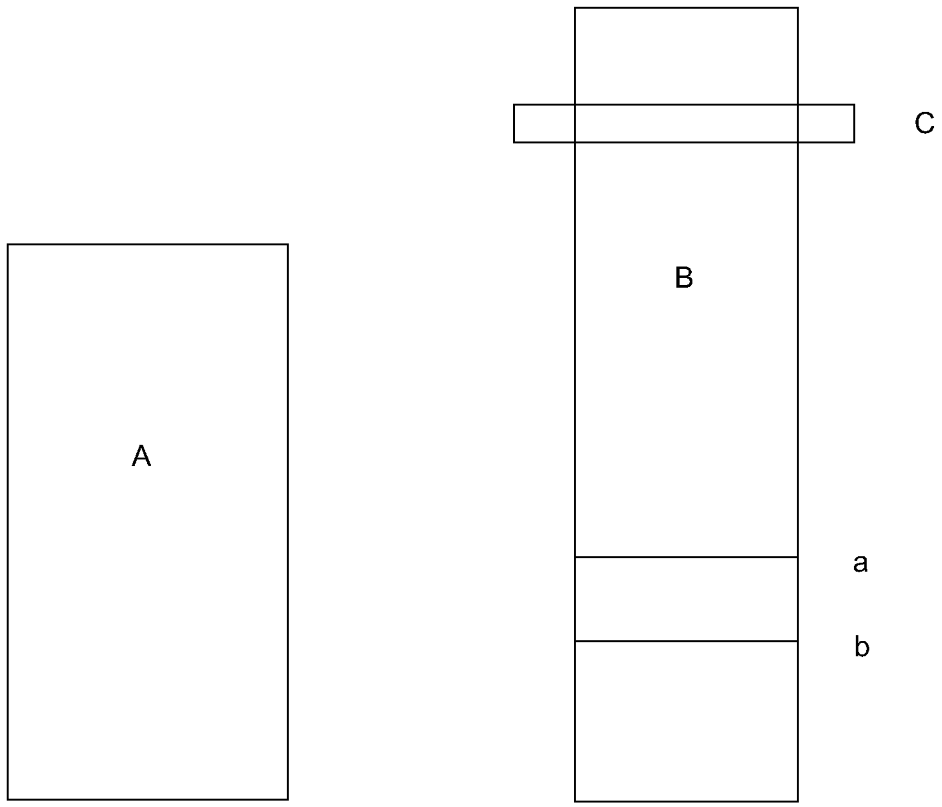

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 372981 A
- EP 574260 A
- US 5684106 A
- WO 2004024816 A
- WO 2005080479 A
- WO 0022018 A
- WO 0053644 A
- WO 0053664 A
- WO 0220068 A
- WO 0222717 A
- EP 530438 A
- EP 547847 A
- EP 559476 A
- EP 632068 A
- WO 9321237 A
- WO 03104299 A
- WO 03104300 A
- WO 03104301 A
- DE 10331450 A
- DE 10331456 A
- DE 10355401 A
- DE 19543368 A
- DE 19646484 A
- WO 9015830 A
- WO 0232962 A
- EP 343427 A
- DE 10319462 A
- DE 19941423 A
- EP 686650 A
- WO 0145758 A
- WO 0138402 A
- DE 3825366 A
- US 6241928 B
- EP 083022 A
- EP 543303 A
- EP 937736 A
- DE 10204938 A
- US 6239230 B
- EP 1199327 A
- DE 4020780 A
- DE 198075022 A
- DE 19807992 A
- DE 198545732 A
- DE 19854574 A
- DE 10204937 A
- DE 10334584 A
- WO 03031482 A


### In der Beschreibung aufgeführte Nicht-Patentliteratur


- **F.L. BUCHHOLZ ; A.T. GRAHAM.** Modern Superabsorbent Polymer Technology. Wiley-VCH, 1998, 69-117